# EUROPEAN PATENT SPECIFICATION

(11) **EP 4 081 499 B1**
(45) Date of publication and mention of the grant of the patent: **09.08.2023**
(21) Application number: 20824929.2
(22) Date of filing: 15.12.2020
(51) Int. Cl.: C07C 29/03, C07C 33/02, C07C 403/08

(54) **FUNCTIONALISATION OF 1,3-ALPHA-DIENES (II)**
FUNKTIONALISIERUNG VON 1,3-ALPHA-DIENEN (II)
FONCTIONNALISATION DE 1,3-ALPHA-DIÈNES (II)

(30) Priority: 23.12.2019 EP 19219363
(43) Date of publication of application: 02.11.2022
(73) Proprietor: DSM IP Assets B.V., 6411 TE Heerlen (NL)
(72) Inventor: BONRATH, Werner, 4303 Kaiseraugst (CH); IMBERI, Felix, 4303 Kaiseraugst (CH); MUELLER, Marc-André, 4303 Kaiseraugst (CH); WUESTENBERG, Bettina, 4303 Kaiseraugst (CH)
(74) Representative: Kurt, Manfred
(86) International application number: PCT/EP2020/086177
(87) International publication number: WO 2021/130059

(56) References cited:
- DE-B- 1 235 309
- JP-B1- S4 920 163
- US-A- 2 990 422

## Description

The present invention relates to the functionalisation of specific 1,3-alpha-dienes. These functionalized 1,3-alpha-dienes are important intermediates in organic synthesis (especially in the synthesis of carotenoids, vitamin A and/or vitamin A derivatives). The goal was to find intermediates in the synthesis of carotenoids, vitamin A and/or vitamin A derivatives and an easy and effective way to produce them.

The present invention relates to the functionalisation of specific 1,3-alpha-dienes by hydroboration followed by an oxidation to the corresponding alcohols. Hydroboration is a well-known reaction from the prior art.

The obtained product, which is a terminal alcohol is a very interesting intermediate in the organic synthesis, especially in the synthesis of carotenoids, vitamin A and vitamin A derivatives. The preparation of such compounds from ester precursors is described in e.g. DE1235309B or JPS4920163B1.

Therefore, there is a need for a hydroboration process, which allows to produce specific and important terminal alcohols in an excellent yield.

Therefore, the present invention relates to a process (P), wherein a compound of formula (I) 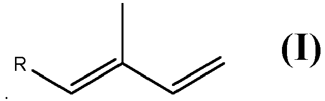 wherein R is 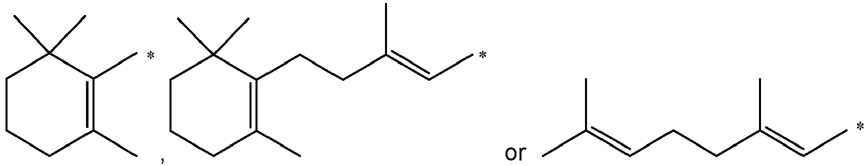 (wherein the asterix shows the connecting bond) is reacted with a borane tetrahydrofuran complex and then oxidized to the corresponding alcohol.

The obtained alcohol has the following formula (II) 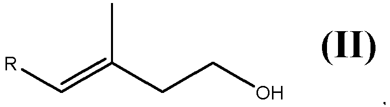 wherein R has the same means as in compound of formula (I).

The hydroboration can be carried out with or without any solvent. In case a solvent is used, the solvent needs to be inert. Usually THF is used or a mixture of THF with at least one other inert solvent (such as i.e. cyclohexene).

Preferably, the hydroboration is carried in an inert solvent.

Therefore, the present invention also relates to a hydroboration process (P1), which is the hydroboration process (P), wherein the process is carried out in an inert solvent.

Therefore, the present invention also relates to a hydroboration process (P1'), which is the hydroboration process (P1), wherein the process is carried out in THF and optionally at least one other solvent.

Preferred is a process wherein the compound of formula (Ia) 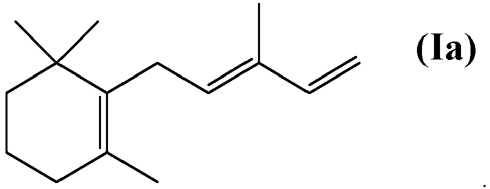 is used as starting material.

Also preferred is a process wherein the compound of formula (Ib) 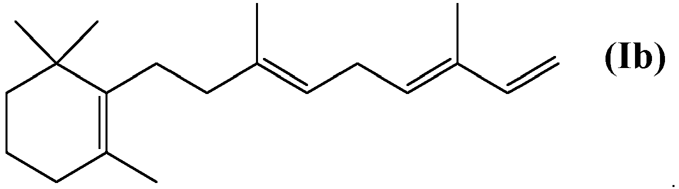 is used as starting material.

Also preferred is a process wherein the compound of formula (Ic) 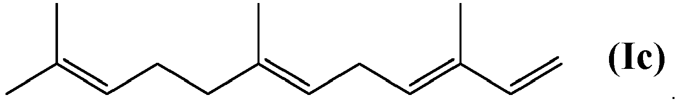 is used as starting material.

Therefore, the present invention also relates to a hydroboration process (P2), which is the hydroboration process (P), (P1) or (P1'), wherein the compound of formula (Ia) 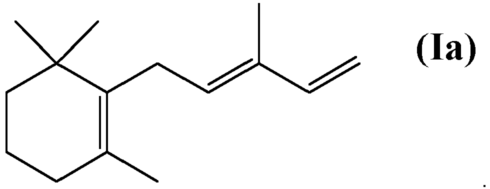 is used as starting material.

Therefore, the present invention also relates to a hydroboration process (P3), which is the hydroboration process (P), (P1) or (P1'), wherein the compound of formula (Ib) 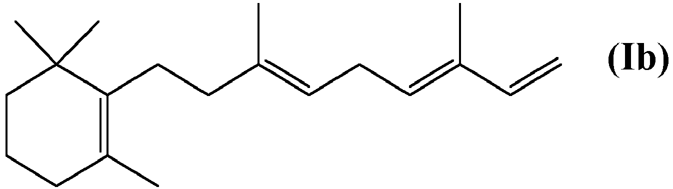 is used as starting material.

Therefore, the present invention also relates to a hydroboration process (P4), which is the hydroboration process (P), (P1) or (P1'), wherein the compound of formula (Ic) 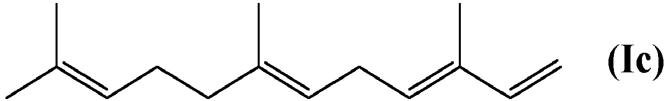 is used as starting material.

Furthermore, the compound of formula (IIb), which is obtained alcohol when the compound formula (Ib) is used as starting material, 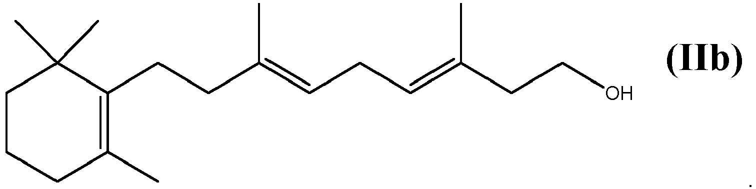 is new.

Therefore, the present invention also relates to the compound of formula (Ilb)

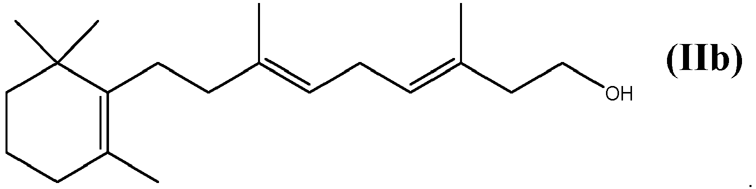

The starting material used in the process according to the present invention can be obtained by commonly known processes.

Cyclo-alpha-farnesene (compound of formula (Ia) can be prepared by literature-known procedures (Desai, Shailesh R. et al, Tetrahedron 1992, 48(3), 481-90) in 52.5% over-all yield.

This is done according to the following way:

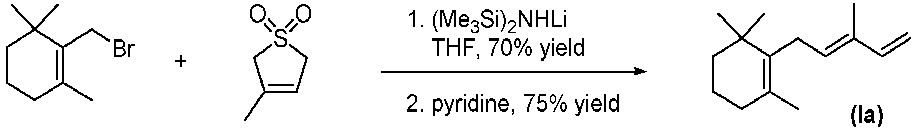

Alternatively, the compound of formula (Ia) can be produced by using the commercially available (2E)-2-methyl-4-(2,6,6-trimethyl-1-cyclohexen-1-yl)-2-butenal via Wittig reaction:

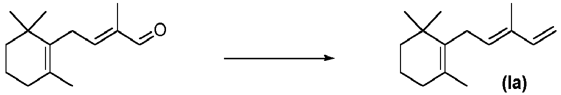

The compound of formula (Ib), which is not known from any prior art, can be produced as follows:

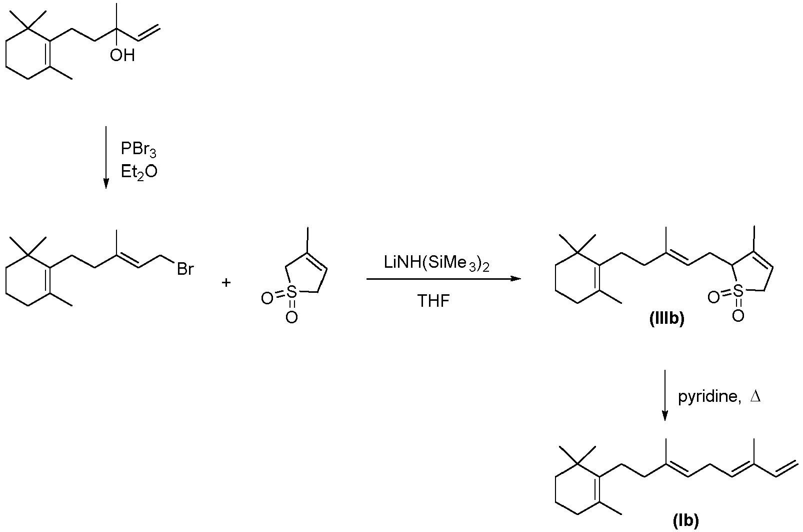

The compound of formula (IIIb) is also a new compound.

The compound of formula (Ic) is produced as follows (according to Synth. Commun. 1990, 20(4), 523-533; J. Agric. Food Chem. 2016, 64, 6809-6818):

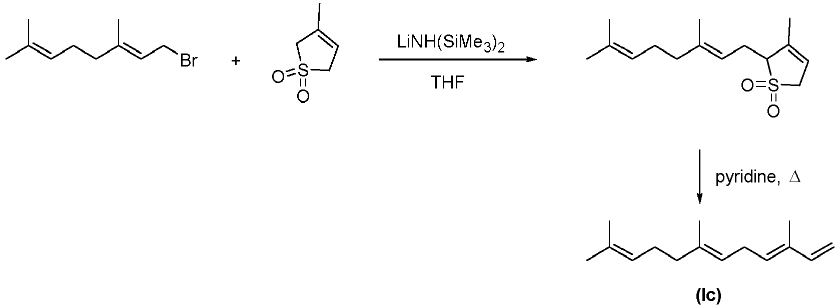

The borane tetrahydrofuran complex (BH3-THF complex or borane-tetrahydrofuran), which is commercially available, is added to the reaction mixture in equimolar (or slight excess) in regard to the compound of formula (I). Preferably, the borane tetrahydrofuran complex is added in a slight excess (between 1.1 - 2 eq) in regard to the compound of formula (I).

Therefore, the present invention also relates to a hydroboration process (P5), which is the hydroboration process (P), (P1), (P1'), (P2), (P3) or (P4), wherein the borane tetrahydrofuran complex is added to the reaction mixture in an equimolar amount in regard to the compound of formula (I).

Therefore, the present invention also relates to a hydroboration process (P5'), which is the hydroboration process (P), (P1), (P1'), (P2), (P3) or (P4), wherein the borane tetrahydrofuran complex is added in a slight excess (between 1.1 - 2 eq) in regard to the compound of formula (I).

The hydroboration reaction is usually carried out at temperature range of from -10°C - 30 °C. Preferred are elevated temperatures (from -5°C to 25°C).

Therefore, the present invention also relates to a hydroboration process (P6), which is the hydroboration process (P), (P1), (P1'), (P2), (P3), (P4), (P5) or (P5'), wherein the process is carried out at temperature range of from -10°C - 30 °C.

Therefore, the present invention also relates to a hydroboration process (P6'), which is the hydroboration process (P6), wherein the process is carried out at temperature range of from -5°C to 25°C.

Furthermore, the hydroboration reaction can be carried out under an inert gas atmosphere (usually N₂ gas).

Therefore, the present invention also relates to a hydroboration process (P7), which is the hydroboration process (P), (P1), (P1'), (P2), (P3), (P4), (P5), (P5'), (P6) or (P6'), wherein the process is carried out under an inert gas atmosphere (usually N₂ gas). To obtain the intermediates, which are very suitable in the organic synthesis (especially in the production of carotenoids, vitamin A and vitamin A derivatives, the reaction products of the hydroboration process (the compounds of formula (III) and (III')) are converted into alcohols via an oxidative cleavage .

The oxidative cleavage is carried according to well-known processes. Usually and preferably the oxidative cleavage is carried in the presence of hydrogen peroxide and a base.

Therefore the present invention also relates to a hydroboration process (P8), which is the hydroboration process (P), (P1), (P1'), (P2), (P3), (P4), (P5), (P5'), (P6) or (P6') or (P7), wherein in a second step the reaction product is converted into the alcohols via an oxidative cleavage in the presence of hydrogen peroxide and a base.

The following example illustrate the invention. All parts are related to weight and the temperatures are given in °C.

### Examples

### Example 1:

In a 25-ml flask under inert gas atmosphere, borane tetrahydrofuran complex (1M, 1.666 ml, 1.666 mmol) was cooled to 0 °C. Cyclohexene (0.169 ml, 1.666 mmol) in THF dry (4.00 ml) was added within 5 min. After 10 min a turbid white reaction mixture was obtained and stirring was continued for 1h at 0°C. Then, cyclo-α-farnesene (compound of formula (Ia)) (200 mg, 0.833 mmol) in THF dry (2.00 ml) was added. The reaction mixture was allowed to warm to room temperature and was monitored by HPLC. After 1h 15min full conversion was observed. Subsequently sodium hydroxide (5.00 ml, 9.99 mmol) and hydrogen peroxide (30%, 0.595 ml, 5.83 mmol) were added and stirring was continued for another hour. Then, the reaction mixture was diluted with diethyl ether (20 ml), transferred to a separation funnel and washed with deionized water. The layers were separated, and the organic layer was washed with brine. The aqueous layers were re-extracted with diethyl ether. The combined organic layers were dried over magnesium sulfate, filtered and evaporated under reduced pressure. The crude product (compound of formula (IIa)) was obtained as colorless oil (263 mg, content by qNMR: 56.4 wt%, 100% conversion, 80.0% yield).

### Example 2:

In a 25-ml flask under inert gas atmosphere, borane tetrahydrofuran complex (1M, 1.869 ml, 1.869 mmol) was cooled to 0 °C. Cyclohexene (0.190 ml, 1.869 mmol) in THF dry (4.50 ml) was added within 5 min. After 10 min a turbid white reaction mixture was obtained and stirring was continued for 1h at 0°C. Then, α-farnesene (compound of formula (Ic)) (200 mg, 0.935 mmol) in THF dry (2.25 ml) was added. The reaction mixture was allowed to warm to room temperature. After 2.5 hours, subsequently sodium hydroxide (5.61 ml, 11.22 mmol) and hydrogen peroxide (30%, 0.668 ml, 6.54 mmol) were added and stirring was continued for another hour. Then, the reaction mixture was diluted with diethyl ether (20 ml), transferred to a separation funnel and washed with deionized water. The layers were separated, and the organic layer was washed with brine. The aqueous layers were re-extracted with diethyl ether. The combined organic layers were dried over magnesium sulfate, filtered and evaporated under reduced pressure. The crude product (compound of formula (IIc)) was obtained as colorless oil (416 mg, content by qNMR: 42.0 wt%, 91.9% conversion, 84.0% yield).

### Example 3:

In a 25-ml flask under inert gas atmosphere, borane tetrahydrofuran complex (1M, 2.433 ml, 2.433 mmol) was cooled to 0 °C. Cyclohexene (0.345 ml, 3.41 mmol) in THF dry (6.25 ml) was added within 5 min. After 15 min a turbid white reaction mixture was obtained and stirring was continued for 1h at 0°C. Then, 1,3-diene (compound or formula (lb)) (500 mg, 1.703 mmol) in THF dry (2.50 ml) was added. The reaction mixture was allowed to warm to room temperature. After 1.5 hours, subsequently sodium hydroxide (5.11 ml, 10.22 mmol) and hydrogen peroxide (30%, 0.609ml, 5.96 mmol) were added and stirring was continued for another hour. Then, the reaction mixture was diluted with diethyl ether (20 ml), transferred to a separation funnel and washed with deionized water. The layers were separated, and the organic layer was washed with brine. The aqueous layers were re-extracted with diethyl ether. The combined organic layers were dried over magnesium sulfate, filtered and evaporated under reduced pressure. The crude product (compound of formula (IIb) was obtained as colorless oil (892 mg, content by qNMR: 36.4 wt%, 90.3% conversion, 66.4% yield).

## Claims

1. A process, wherein a compound of formula (I) 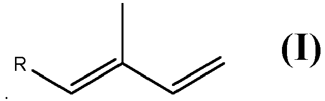 wherein R is 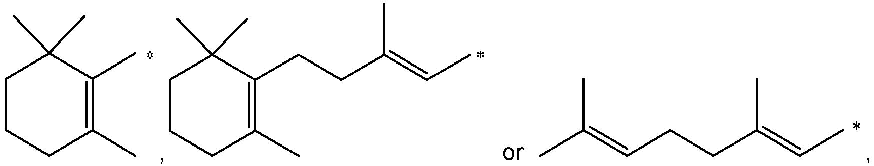 wherein the asterix shows the connecting bond, is reacted with a borane tetrahydrofuran complex and then oxidized to the corresponding alcohol.

2. Process according to claim 1, wherein the process is carried out in an inert solvent.

3. Process according to claim 1, wherein the process is carried out in THF and optionally at least one other solvent.

4. Process according to any of the preceding claims, wherein the compound of formula (Ia) 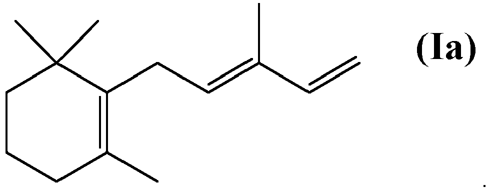 is used as starting material.

5. Process according to any of the preceding claims 1 - 3, wherein the compound of formula (Ib) 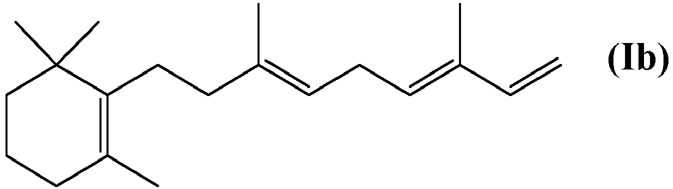 is used as starting material.

6. Process according to any of the preceding claims 1 - 3, wherein the compound of formula (Ic) 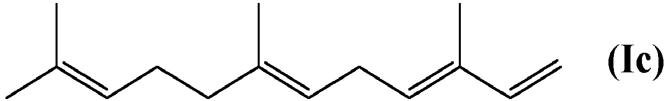 is used as starting material.

7. Process according to any of the preceding claims, wherein the borane tetrahydrofuran complex is added to the reaction mixture in an equimolar amount in regard to the compound of formula (I).

8. Process according to any of the preceding claims 1 - 6, wherein the borane tetrahydrofuran complex is added in a slight excess, between 1.1 - 2 eq., in regard to the compound of formula (I).

9. Process according to any of the preceding claims, wherein the process is carried out at temperature range of from -10°C - 30 °C.

10. Process according to any of the preceding claims 1 - 8, wherein the process is carried out at temperature range of from -5°C to 25°C.

11. Process according to any of the preceding claims, wherein the process is carried out under an inert gas atmosphere, usually N₂ gas.

12. Process according to any of the preceding claims, wherein in a second step the reaction product is converted into the alcohols via an oxidative cleavage in the presence of hydrogen peroxide and a base.

13. Compound of formula (Ilb)

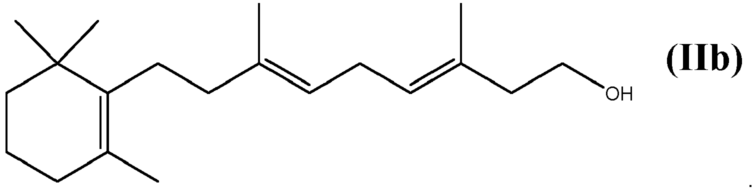

## Patentansprüche

1. Verfahren, bei dem eine Verbindung der Formel (I) 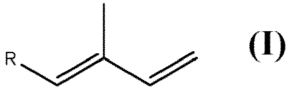 wobei R für 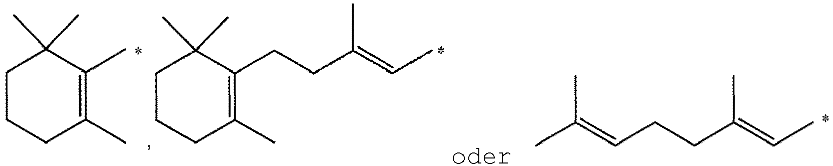 steht, wobei das Sternchen die verknüpfende Bindung anzeigt, mit einem Boran-Tetrahydrofuran-Komplex umsetzt und dann zu dem entsprechenden Alkohol oxidiert.

2. Verfahren nach Anspruch 1, wobei das Verfahren in einem inerten Lösungsmittel durchgeführt wird.

3. Verfahren nach Anspruch 1, wobei das Verfahren in THF und gegebenenfalls mindestens einem anderen Lösungsmittel durchgeführt wird.

4. Verfahren nach einem der vorhergehenden Ansprüche, wobei die Verbindung der Formel (Ia) 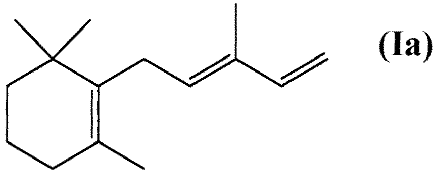 als Ausgangsstoff verwendet wird.

5. Verfahren nach einem der Ansprüche 1-3, wobei die Verbindung der Formel (Ib) 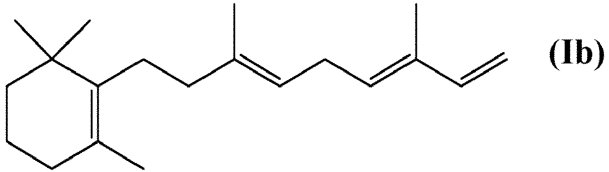 als Ausgangsstoff verwendet wird.

6. Verfahren nach einem der Ansprüche 1-3, wobei die Verbindung der Formel (Ic) 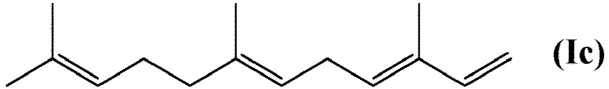 als Ausgangsstoff verwendet wird.

7. Verfahren nach einem der vorhergehenden Ansprüche, wobei der Boran-Tetrahydrofuran-Komplex in einer äquimolaren Menge in Bezug auf die Verbindung der Formel (I) zur Reaktionsmischung gegeben wird.

8. Verfahren nach einem der vorhergehenden Ansprüche 1-6, wobei der Boran-Tetrahydrofuran-Komplex in einem leichten Überschuss, zwischen 1,1-2 Äq. in Bezug auf die Verbindung der Formel (I) zugegeben wird.

9. Verfahren nach einem der vorhergehenden Ansprüche, wobei das Verfahren in einem Temperaturbereich von -10 °C-30 °C durchgeführt wird.

10. Verfahren nach einem der vorhergehenden Ansprüche 1-8, wobei das Verfahren in einem Temperaturbereich von -5 °C bis 25 °C durchgeführt wird.

11. Verfahren nach einem der vorhergehenden Ansprüche, wobei das Verfahren unter Inertgasatmosphäre, üblicherweise N₂-Gas, durchgeführt wird.

12. Verfahren nach einem der vorhergehenden Ansprüche, wobei das Reaktionsprodukt in einen zweiten Schritt durch oxidative Spaltung in Gegenwart von Wasserstoffperoxid und einer Base in die Alkohole umgewandelt wird.

13. Verbindung der Formel (IIb)

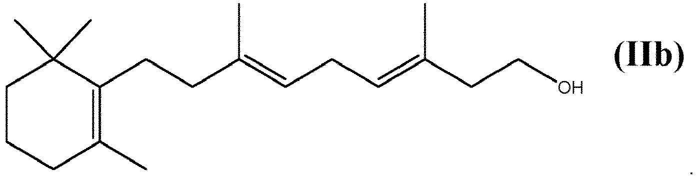

## Revendications

1. Procédé, dans lequel un composé de formule (I) 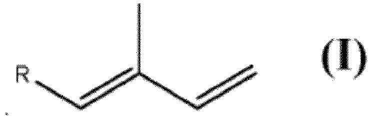 R étant 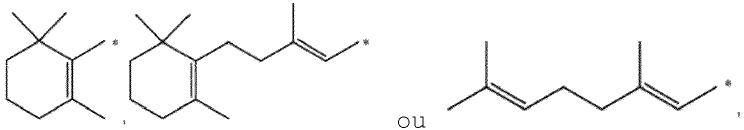 l'astérisque montrant la liaison de connexion,
est mis à réagir avec un complexe de borane tétrahydrofuranne et ensuite oxydé en l'alcool correspondant.

2. Procédé selon la revendication 1, le procédé étant mis en œuvre dans un solvant inerte.

3. Procédé selon la revendication 1, le procédé étant mis en œuvre dans du THF et éventuellement au moins un autre solvant.

4. Procédé selon l'une quelconque des revendications précédentes, le composé de formule (Ia) 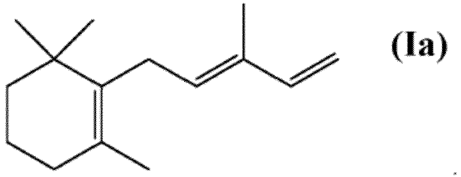 étant utilisé en tant que matière de départ.

5. Procédé selon l'une quelconque des revendications précédentes 1 à 3, le composé de formule (Ib) 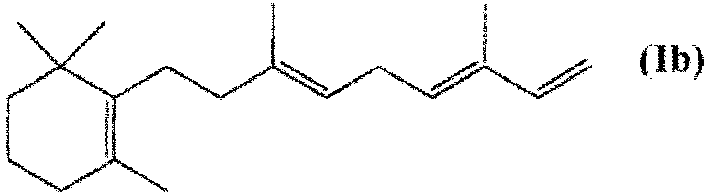 étant utilisé en tant que matière de départ.

6. Procédé selon l'une quelconque des revendications précédentes 1 à 3, le composé de formule (Ic) 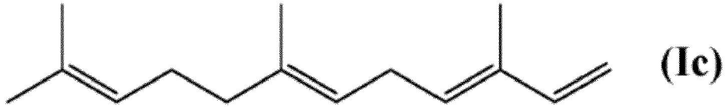 étant utilisé en tant que matière de départ.

7. Procédé selon l'une quelconque des revendications précédentes, le complexe de borane tétrahydrofuranne étant ajouté au mélange réactionnel en une quantité équimolaire par rapport au composé de formule (I).

8. Procédé selon l'une quelconque des revendications précédentes 1 à 6, le complexe de borane tétrahydrofuranne étant ajouté en un léger excès, entre 1,1 et 2 éq. par rapport au composé de formule (I).

9. Procédé selon l'une quelconque des revendications précédentes, le procédé étant mis en œuvre à une plage de températures allant de -10 °C à 30 °C.

10. Procédé selon l'une quelconque des revendications précédentes 1 à 8, le procédé étant mis en œuvre à une plage de températures allant de -5 °C à 25 °C.

11. Procédé selon l'une quelconque des revendications précédentes, le procédé étant mis en œuvre sous une atmosphère de gaz inerte, habituellement du gaz de N₂.

12. Procédé selon l'une quelconque des revendications précédentes, dans lequel dans une deuxième étape, le produit de réaction est converti en les alcools via un clivage oxydant en la présence de peroxyde d'hydrogène et d'une base.

13. Composé de formule (IIb)
